# EUROPEAN PATENT APPLICATION

(11) **EP 4 239 361 A1**
(43) Date of publication of application: **06.09.2023**
(21) Application number: 21892133.6
(22) Date of filing: 30.09.2021
(51) Int. Cl.: G01S 5/02, G01S 13/02, G01S 5/14, G01S 5/04, G01S 13/89, G01S 7/04, G01S 7/292, A61B 5/024, A61B 5/08, A61B 5/05

(54) **ELECTRONIC APPARATUS AND OPERATING METHOD THEREFOR**

(30) Priority: 10.11.2020 KR 20200149594
(71) Applicant: Samsung Electronics Co., Ltd., Suwon-si, Gyeonggi-do 16677 (KR)
(72) Inventor: KANG, Kunsok, Suwon-si, Gyeonggi-do 16677 (KR); SOH, Byungseok, Suwon-si, Gyeonggi-do 16677 (KR); YOON, Eungsik, Suwon-si, Gyeonggi-do 16677 (KR)
(74) Representative: Walaski, Jan Filip
(86) International application number: PCT/KR2021/013463
(87) International publication number: WO 2022/102963

(57) **Abstract**

Provided is an operating method of an electronic apparatus. The operating method of the electronic apparatus, performed using a UWB communication module including a plurality of antennas, includes emitting a UWB transmission signal, receiving a UWB reflection signal reflected from an object, and obtaining three-dimensional information of the object based on the UWB transmission signal and the UWB reflection signal, wherein the plurality of antennas are respectively arranged in a vertical direction and a horizontal direction on a front surface of a display of the electronic apparatus.

## Description

### Technical Field

Various embodiments disclosed herein relate to an electronic apparatus and an operating method thereof, and more particularly, to an electronic apparatus for three-dimensionally detecting a movement of a user through wireless communication, and an operating method thereof.

### Background Art

Ultra-wideband (UWB) refers to a wireless technology in which the occupied frequency is 20 % or more of a central frequency or the occupied bandwidth is 500 MHz or more. With UWB, a distance to a target may be measured using a very broad frequency band compared to existing spectrums.

Impulse radio ultra-wideband (IR-UWB) radar technology is a short distance measurement technology where an impulse signal is transmitted or received using a broadband frequency. The IR-UWB radar technology has low power consumption, high distance resolution, and high penetrability, and is used in various fields.

### Disclosure

### Technical Solution

An operating method of an electronic apparatus according to an embodiment, performed using a UWB module including a plurality of antennas, includes emitting a UWB transmission signal, receiving a UWB reflection signal reflected from an object, and obtaining three-dimensional information of the object based on the UWB transmission signal and the UWB reflection signal, wherein the plurality of antennas may be respectively arranged in a vertical direction and a horizontal direction on a front surface of a display of the electronic apparatus.

### Description of Drawings

FIG. 1 is a diagram illustrating an ultra-wideband (UWB) module mounted in an electronic apparatus, according to an embodiment.
FIG. 2 is an internal block diagram of an electronic apparatus according to an embodiment.
FIG. 3 is a diagram for describing obtaining of information about an object by using an antenna included in a UWB module, according to an embodiment.
FIG. 4 is a diagram for describing obtaining of three-dimensional information of an object by using an arrangement of a plurality of antennas included in a UWB module, according to an embodiment.
FIG. 5 is a diagram for describing an electronic apparatus detecting an object, according to an embodiment.
FIG. 6 is a diagram illustrating a signal reflected from an object, according to an embodiment.
FIG. 7 is a diagram for describing removal of noise from three-dimensional information of an object, according to an embodiment.
FIG. 8 is a graph showing a bio-signal from which movement noise is removed, according to an embodiment.
FIG. 9 is an internal block diagram of an electronic apparatus according to an embodiment.
FIG. 10 is a diagram for describing an electronic apparatus outputting comparison content, according to an embodiment.
FIG. 11 is a diagram for describing a case where an electronic apparatus has detected an abnormal change in a bio-signal, according to an embodiment.
FIG. 12 is a flowchart of an operating method of an electronic apparatus, according to an embodiment.
FIG. 13 is a flowchart of an operating method of an electronic apparatus, according to an embodiment.
FIG. 14 is a flowchart of an operating method of an electronic apparatus, according to an embodiment.

### Mode for Invention

In an embodiment, the obtaining of the three-dimensional information of the object may include obtaining the three-dimensional information of the object by using at least one of positions of the plurality of antennas, an emission time of the UWB transmission signal, a reception time of the UWB reflection signal, and magnitudes and angles of the UWB transmission signal and the reflection signal.

In an embodiment, the obtaining of the three-dimensional information of the object may include obtaining at least one of a difference in emission times of the UWB transmission signal between the plurality of antennas, a difference in reception times of the UWB reflection signal between the plurality of antennas, a difference in magnitudes and angles of the UWB transmission signal and the UWB reception signal between the plurality of antennas, and a difference in the positions of the plurality of antennas.

In an embodiment, the three-dimensional information of the object may include at least one of position information, direction information, movement amount information, and movement speed information of the object.

In an embodiment, the method may further include obtaining a bio-signal from which movement noise is removed, from the three-dimensional information of the object, wherein the obtaining of the bio-signal from which the movement noise is removed incudes obtaining a signal on a frequency domain, from the three-dimensional information of the object, filtering a signal having a certain frequency range, from the signal on the frequency domain, and inversely transforming the filtered signal having the certain frequency range into a time domain.

In an embodiment, the obtaining of the signal on the frequency domain may include obtaining a signal of each of an X-axis, a Y-axis, and a Z-axis from the three-dimensional information of the object, and converting the signal of each of the X-axis, the Y-axis, and the Z-axis into a frequency domain signal.

In an embodiment, the bio-signal may include at least one of a heart rate and respiration.

In an embodiment, the method may further include outputting the bio-signal.

In an embodiment, the method may further include detecting a change in the bio-signal, the change being greater than or equal to a reference value, generating notification information in response to the change in the bio-signal, the change being greater than or equal to the reference value, and outputting the notification information.

In an embodiment, the notification information may include at least one of an audio signal and a video signal, and the outputting of the notification information may include at least one of outputting the notification information through the electronic apparatus and transmitting the notification information to an external user terminal through a communication network.

An electronic apparatus according to an embodiment includes a UWB module including a plurality of antennas, a memory storing one or more instructions, and a processor configured to execute the one or more instructions stored in the memory, wherein the UWB module transmits a UWB transmission signal to an object and receives a UWB reflection signal reflected from the object, and the processor executes the one or more instructions to obtain three-dimensional information of the object based on the UWB transmission signal and the UWB reflection signal, and the plurality of antennas are respectively arranged in a vertical direction and a horizontal direction on a front surface of the electronic apparatus.

A computer-readable recording medium according to an embodiment may be a computer-readable recording medium having recorded thereon a program for executing an operating method of an electronic apparatus, the operating method being performed using a UWB communication module including a plurality of antennas, and including emitting a UWB transmission signal, receiving a UWB reflection signal reflected from an object, and obtaining three-dimensional information of the object based on the UWB transmission signal and the UWB reflection signal, wherein the plurality of antennas are respectively arranged in a vertical direction and a horizontal direction on a front surface of a display of the electronic apparatus.

Hereinafter, embodiments of the present disclosure will now be described more fully with reference to the accompanying drawings, in which the embodiments of the present disclosure are shown such that one of ordinary skill in the art may easily work the present disclosure. The present disclosure may, however, be embodied in many different forms and should not be construed as limited to the embodiments set forth herein.

The terms used in the present disclosure are those general terms currently widely used in the art in consideration of functions in regard to the present disclosure, but the terms may vary according to the intention of those of ordinary skill in the art, precedents, or new technology in the art. Thus, the terms used in the present disclosure should be understood not as names of the terms but based on the meaning of the terms and the overall description of the present disclosure.

The terminology used in the present disclosure is for the purpose of describing particular embodiments only and is not intended to be limiting of example embodiments.

Throughout the specification, when a portion is "connected" to another portion, the portion is connected to the other portion not only directly but also electrically through another portion interposed therebetween.

As used in the present specification, particularly, in the claims, the singular forms "a," "an" and "the" are intended to include the plural forms as well, unless the context clearly indicates otherwise. Finally, the steps of all methods described herein can be performed in any suitable order unless otherwise indicated herein or otherwise clearly contradicted by context. The present disclosure is not limited by the order of description of the described steps.

The expressions "in some embodiments" or "in one embodiment" in various parts in this specification are not necessarily all referring to the same embodiment.

Some embodiments of the present disclosure may be described in terms of functional block components and various processing steps. Such functional blocks may be realized by any number of hardware and/or software components configured to perform the specified functions. For example, functional blocks of the present disclosure may be implemented using one or more microprocessors or by circuit components for certain functions. Also, for example, the functional blocks of the present disclosure may be implemented using various programming or scripting languages. Functional blocks may be implemented in algorithms that execute on one or more processors. Furthermore, the present disclosure could employ any number of conventional techniques for electronics configuration, signal processing and/or data processing and the like. The words "mechanism" and "element" are used broadly and are not limited to mechanical or physical components.

Furthermore, the connecting lines, or connectors shown in the various figures presented are intended to represent exemplary functional relationships and/or physical or logical couplings between the various elements. It should be noted that many alternative or additional functional relationships, physical connections or logical connections may be present in a practical device.

Also, in the specification, the term "... units" or "... modules" denote units or modules that process at least one function or operation, and may be realized by hardware, software, or a combination of hardware and software.

Also, in the specification, the term "user" refers to a person who uses an electronic apparatus to control a function or operation of the electronic apparatus, and may include a viewer, a consumer, a manager, or an installation engineer. Also, in the specification, the term "object" refers to an object that an electronic apparatus detects by using a UWB module, and a user using the electronic apparatus may be an object.

The present disclosure will now be described in detail with reference to the attached drawings.

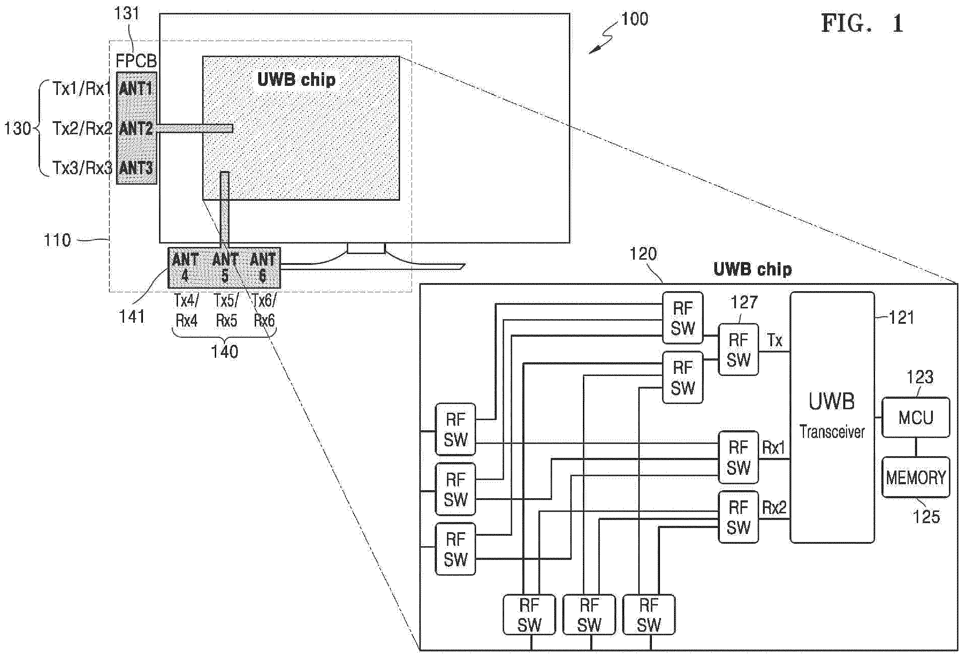

FIG. 1 is a diagram illustrating an ultra-wideband (UWB) module mounted in an electronic apparatus 100, according to an embodiment.

Referring to FIG. 1, the electronic apparatus 100 may include a UWB module 110.

In an embodiment, the electronic apparatus 100 may be an image display apparatus. The electronic apparatus 100 may be a digital TV capable of receiving digital broadcasting, but is not limited thereto, and may be implemented in various types of electronic apparatuses in which a UWB module may be included.

For example, the electronic apparatus 100 may include at least one of a desktop, a smartphone, a tablet personal computer (PC), a mobile phone, a video phone, an e-book reader, a laptop PC, a netbook computer, a digital camera, a Personal Digital Assistants (PDA), a Portable Multimedia Player (PMP), a camcorder, a navigation device, a wearable device, a smart watch, a home network system, a security system, and a medical device.

In an embodiment, the UWB module 110 included in the electronic apparatus 100 may include a UWB chip 120, FPCBs 131 and 141, and a plurality of antennas 130 and 140 formed on the FPCBs 131 and 141.

When the electronic apparatus 100 is an image display apparatus, a bezel of the image display apparatus tends to become thinner. Thus, it is necessary to mount the UWB module 110 in the electronic apparatus 100 in consideration of a width of the bezel.

In an embodiment, as illustrated in FIG. 1, the plurality of antennas 130 and 140 among components included in the UWB module 110 may be placed on a front surface of the electronic apparatus 100, and the UWB chip 120 may be placed on a rear surface of the electronic apparatus 100. The plurality of antennas 130 and 140 are patterned antennas and may be mounted on the FPCBs 131 and 141. A printed circuit board (PCB) is a circuit component capable of transmitting electrical signals, and a flexible printed circuit board (FPCB) is an electrical circuit board which is a hard PCB to which flexible characteristics. to which are imparted.

In an embodiment, in order to minimize the size of the UWB module 110, the UWB chip 120 is placed on the rear surface of the electronic apparatus 100, and the UWB chip 120 and the FPCBs 131 and 141 may be connected to each other and the FPCBs 131 and 141 are folded and placed on a front surface of a display, and then the plurality of antennas 130 and 140 may be mounted on the FPCBs 131 and 141 in a pattern form. Accordingly, the plurality of antennas 130 and 140 may be located near the bezel, and the UWB chip 120 may be connected to a main body of the electronic apparatus 100 on the rear surface of the electronic apparatus 100.

In an embodiment, the FPCBs 131 and 141 may be arranged in vertical and horizontal directions of the electronic apparatus 100, respectively. The plurality of antennas 130 and 140 may be mounted on the FPCBs 131 and 141 arranged in the vertical and horizontal directions of the electronic apparatus 100, respectively. As the plurality of antennas 130 and 140 are arranged in the vertical and horizontal directions on the front surface of the electronic apparatus 100, respectively, three-dimensional information such as a position, a movement, and a direction of an object may be obtained.

For example, as illustrated in FIG. 1, antennas ANT1, ANT2, and ANT3 130 in a vertical direction may be mounted on the FPCB 131 arranged in a direction perpendicular to the front surface of the display of the electronic apparatus 100, that is, in a vertical direction, and antennas ANT4, ANT5, and ANT6 140 in a horizontal direction may be mounted on the FPCB 141 arranged in a horizontal direction to the front surface of the display of the electronic apparatus 100, that is, in a breadthwise direction. Here, the number of antennas 130 in the vertical and horizontal directions may be plural in each direction.

In an embodiment, each antenna may be one of an RX antenna, a TX antenna, or a combined TX/RX antenna. Tx refers to a transmission path, Rx refers to a reception path, and a TX antenna refers to a transmission antenna performing a function of transmitting a wireless signal, and an RX antenna may refer to a reception antenna performing a function of receiving a wireless signal. A combined TX/RX antenna may refer to a transmission/reception antenna in which a single antenna performs both transmission and reception functions.

An RF switch 127 may be a switch circuit that determines whether to pass or block an RF signal. The RF switch 127 may switch an RF signal path depending on whether to transmit or receive a signal by using a corresponding antenna.

The UWB chip 120 may include a UWB transceiver 121, an MCU 123, a memory 125, and the RF switch 127.

The UWB transceiver 121 is a device for transmitting and receiving data by using UWB communication, and may perform functions of a transmitter for transmitting data and a receiver for receiving data. The UWB transceiver 121 is connected to the plurality of antennas 130 and 140 through the RF switch 127 to simultaneously perform data transmission and reception. The UWB transceiver 121 may be connected to the MCU 123 by using a Serial Peripheral Interface (SPI) or the like. The SPI may be one of serial communication methods such as I2C, CAN, and UART.

The micro controller unit (MCU) 123 is a dedicated processor of the UWB module 110 and may control the UWB module 110 overall.

The MCU 123 may process transmission and reflection signals of a UWB signal through the plurality of antennas 130 and 140 and the UWB transceiver 121.

By controlling the UWB module 110 by executing at least one instruction or program stored in the memory 125, the MCU 123 may enable the UWB module 100 to operate. Also, in an embodiment, the MCU 123 may detect at least one of a position, a distance, a movement, and a direction of an object by using a UWB signal transmitted and received through the UWB transceiver 121.

However, this is an embodiment, and a processor (not shown) of the electronic apparatus 100, not the MCU 123, may process a UWB signal transmitted and received through the UWB transceiver 121 and obtain three-dimensional information of the object.

The memory 125 may be, for example, a flash type memory. The memory 125 may store at least one instruction or program executed by the MCU 123.

As illustrated in FIG. 1, each of the plurality of antennas 130 and 140 may be connected to the RF switch 127. In addition, the RF switch 127 connected to each of the plurality of antennas 130 and 140 may be directly connected to the UWB transceiver 121, but may be connected to the UWB transceiver 121 through another RF switch 127. That is, the UWB transceiver 121 may individually drive each of the plurality of antennas 130 and 140 through one RF switch 127 or a combination of a plurality of RF switches 127.

FIG. 1 illustrates an embodiment, in which the UWB transceiver 121 transmits one TX signal to the surroundings through six antennas (ANT1, ANT2, ANT3, ANT4, ANT5, and ANT6) through a combination of a plurality of RF switches 127. In FIG. 1, all six antennas (ANT1, ANT2, ANT3, ANT4, ANT5, and ANT6) are combined TX/RX antennas, and thus, each of the six antennas (ANT1, ANT2, ANT3, ANT4, ANT5, and ANT6) may transmit a transmission signal simultaneously or sequentially with a certain time difference.

A transmission signal emitted by a transmission antenna may collide with objects around the electronic apparatus 100 and be reflected. A reception antenna included in the UWB module 110 may receive a UWB reflection signal reflected from an object.

The UWB chip 120 of FIG. 1 inputs a signal received by the antenna 130 in a vertical direction, to the UWB transceiver 121 as an RX1 signal through a combination of a plurality of RF switches 127, and inputs a signal received by the antenna 140 in a horizontal direction to the UWB transceiver 121 as an RX2 signal, through the combination of the plurality of RF switches 127.

In an embodiment, the electronic apparatus 100 may obtain three-dimensional information of an object based on a UWB transmission signal and a UWB reflection signal.

In an embodiment, a signal reception time of a reflection signal received by each of the plurality of antennas 130 and 140 and an angle between an object and the antennas may vary according to a position of each of the plurality of antennas 130 and 140, a position of the object, and a transmission time of a transmission signal. In an embodiment, the electronic apparatus 100 may obtain three-dimensional information of an object by using a position of each antenna, an emission time of a transmission signal, a reception time of a reflection signal, a magnitude and angle of the transmission signal, a magnitude and angle of the reflection signal, etc.

As such, according to an embodiment, the electronic apparatus 100 may include the UWB module 110 including the plurality of antennas 130 and 140 arranged in the vertical and horizontal directions. The electronic apparatus 100 may emit a UWB transmission signal by using the UWB module 110 and receive a UWB reflection signal reflected from an object. The electronic apparatus 100 may obtain three-dimensional information of an object based on the UWB transmission signal and the UWB reflection signal.

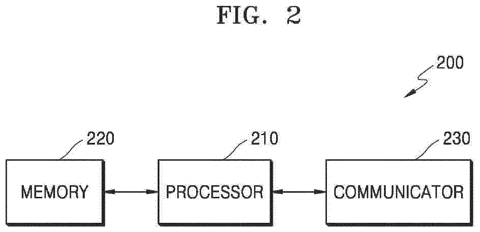

FIG. 2 is an internal block diagram of an electronic apparatus 200 according to an embodiment.

Referring to FIG. 2, the electronic apparatus 200 may include a processor 210, a memory 220, and a communicator 230. The electronic apparatus 200 of FIG. 2 may include the electronic apparatus 100 of FIG. 1.

The communicator 230 may include a UWB module. The UWB module may include a plurality of antennas. The UWB module may emit a UWB transmission signal by using a plurality of transmission antennas and receive a UWB reflection signals reflected from an object by using a plurality of reception antennas.

The UWB module performs the same function as the UWB module 110 described in FIG. 1, and thus, repeated description thereof will be omitted.

The memory 220 may store at least one instruction. The memory 220 may store at least one program executed by the processor 210. Predefined operation rules or programs may be stored in the memory 220. Also, the memory 220 may store data input to or output from the electronic apparatus 200.

The memory 220 may include at least one type of storage medium from among a flash memory type, a hard disk type, a multimedia card micro type, a card type memory (e.g., SD or XD memory, etc.), random access memory (RAM), static random access memory (SRAM), read-only memory (ROM), electrically erasable programmable read-only memory (EEPROM), programmable read-only memory (PROM), magnetic memory, a magnetic disk, or an optical disk.

The processor 210 may control the overall operation of the electronic apparatus 200. In an embodiment, the processor 210 may obtain three-dimensional information of an object based on a UWB transmission signal emitted by the communicator 230 and a UWB reflection signal reflected from the object and received.

In an embodiment, the processor 210 may obtain three-dimensional information of an object by using at least one of positions of a plurality of antennas included in a UWB module in the communicator 230, a time when each of a plurality of transmission antennas emits a UWB transmission signal, a time when each of a plurality of reception antennas receives a UWB reflection signal, magnitudes and angles of the UWB transmission signal and the UWB reflection signal.

In an embodiment, the processor 210 may obtain a difference between signals transmitted and received by each of a plurality of transmission antennas and reception antennas in order to obtain three-dimensional information on an object. That is, the processor 210 may obtain a difference in emission times of a UWB transmission signal between a plurality of transmission antennas, a difference in reception times of a UWB reflection signal between a plurality of reception antennas, a difference in magnitudes and angles of UWB transmission signals between the plurality of transmission antennas, a difference in magnitudes and angles of UWB reception signals between the plurality of reception antennas, and a difference in positions of a plurality of antennas. The processor 210 may obtain three-dimensional information of an object based on the differences between the antennas.

The three-dimensional information of the object may include coordinate values of the object in three dimensions, that is, position information or direction information of the object. Also, the three-dimensional information of the object may include information about an amount of movement or a movement speed of the object on the three-dimensional coordinates.

A UWB signal obtained by using the UWB module may include information about a movement of an object. When the object is a human, the information about the movement of the object may include information about a movement of minute biological organs such as the heartbeat or respiration of the human, in addition to large motions of the human.

In an embodiment, the processor 210 may obtain an accurate bio-signal by removing large motions of the object from a UWB reflection signal reflected from the object and received by using a reception antenna. That is, the processor 210 may remove movement noise other than the bio-signal, from the three-dimensional information of the object.

To this end, the processor 210 may obtain a signal on a frequency domain from the three-dimensional information of the object. The processor 210 may obtain a signal for each axis from the three-dimensional information of the object. The processor 210 may convert each signal of an X-axis, a Y-axis, and a Z-axis obtained from the three-dimensional information of the object into a signal on the frequency domain. The processor 210 may perform, for example, Fast Fourier Transform (FFT) to convert signals for each axis in a time domain into signals in the frequency domain, and filter only signals that are in a frequency range related to bio-signals from the signals in the frequency domain. The bio-signal may include, for example, at least one of a heartbeat signal and a respiration signal.

In an embodiment, the processor 210 may inversely transform a filtered signal back into a signal in the time domain to obtain a bio-signal from which movement noise is removed in the time domain.

In an embodiment, the processor 210 may output a bio-signal.

In an embodiment, the processor 210 may detect a change in a bio-signal, the change being greater than or equal to a reference value. For example, when the heart rate or respiration rate suddenly changes beyond the reference value, the processor 210 may generate notification information indicating the change. The notification information may include at least one of an audio signal and a video signal. The processor 210 may output notification information by using the electronic apparatus 100. In addition, the processor 210 may transmit notification information to a pre-registered external user terminal (not shown) or an institution such as a hospital or fire station through a communication network.

As described above, according to an embodiment, the processor 210 may accurately detect a movement of the object by obtaining three-dimensional information of the object by using the UWB module included in the communicator 230. Also, the processor 210 may obtain a more accurate bio-signal from three-dimensional information of an object.

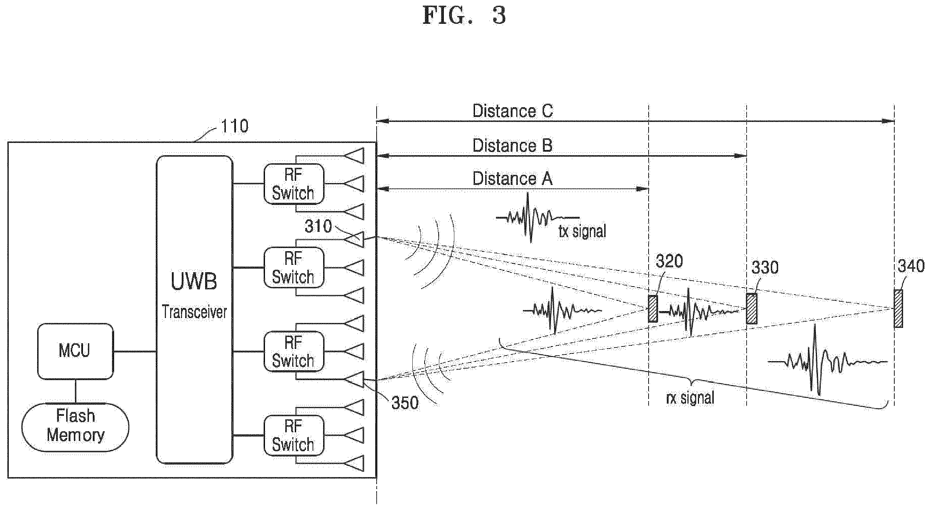

FIG. 3 is a diagram for describing obtaining of information about an object by using an antenna included in a UWB module, according to an embodiment.

Referring to FIG. 3, the UWB module 110 may include a UWB transceiver, an MCU, a memory, an RF switch, and a plurality of antennas.

FIG. 3 illustrates, for convenience of description, that a transmission signal, a to signal, emitted only from one transmission antenna among a plurality of antennas included in the UWB module 110, that is, from the first antenna 310, collides with an object and is reflected.

When three objects near the electronic apparatus 100 in FIG. 3 are respectively referred to as a first object 320, a second object 330, and a third object 340, the first to third objects 310, 320, and 330 are apart from the first antenna 310 by different distances, that is, distances A, B, and C, respectively.

When the first antenna 310 emits a pulse signal tx signal, the signal emitted through the first antenna 310 may collide with the first object 320 and be reflected. At least one reception antenna among the plurality of antennas included in the UWB module 110 may receive a reflected signal. While FIG. 3 illustrates a case where a second antenna 350 receives the reflected signal, according to embodiments, the antennas included in the UWB module 110 may be a combined transmission/reception antenna. For example, a signal emitted from a single combined transmission/reception antenna may be reflected from an object and received through the same combined transmission/reception antenna.

In FIG. 3, when a time point at which the first antenna 310 emits a signal is 0, a signal reflected from the first object 320 may be received by the second antenna 350 after a time of ΔTA. In addition, the signal emitted by the first antenna 310 may collide with the second object 330 and be reflected, and may be received by the second antenna 350 after a time of ΔTB. Similarly, a signal emitted through the first antenna 310 may be reflected from the third object 340 and received by the second antenna 350 after a time of ΔTC.

As illustrated in FIG. 3, signals reflected by the first to third objects 310, 320, and 330, respectively, that is, rx signals, are of different types of signals. That is, signals respectively reflected from the first to third objects 310, 320, and 330 may have varying signal waveforms, for example, signal magnitudes or angles, according to positions of the first antenna 310 and the second antenna 350, a time when the first antenna 310 emits a transmission signal, a time required for the transmission signal to be reflected from an object and reach a reception antenna, and a distance from the antenna to the object. A change in the magnitude or angle of a signal in the time domain may refer to a change in the amplitude or phase of the signal in the frequency domain.

The MCU included in the UWB module 110 or a processor in an electronic apparatus connected to the UWB module 110 may obtain information of an object by using at least one of the positions of the first antenna 310 and the second antenna 350, a time when the first antenna 310 emits a UWB transmission signal, a time when the second antenna 350 receives a UWB reflection signal, a magnitude and angle of the UWB transmission signal, a magnitude and angle of the UWB reflection signal, that is, at least one of an angle between the object and the second antenna 350.

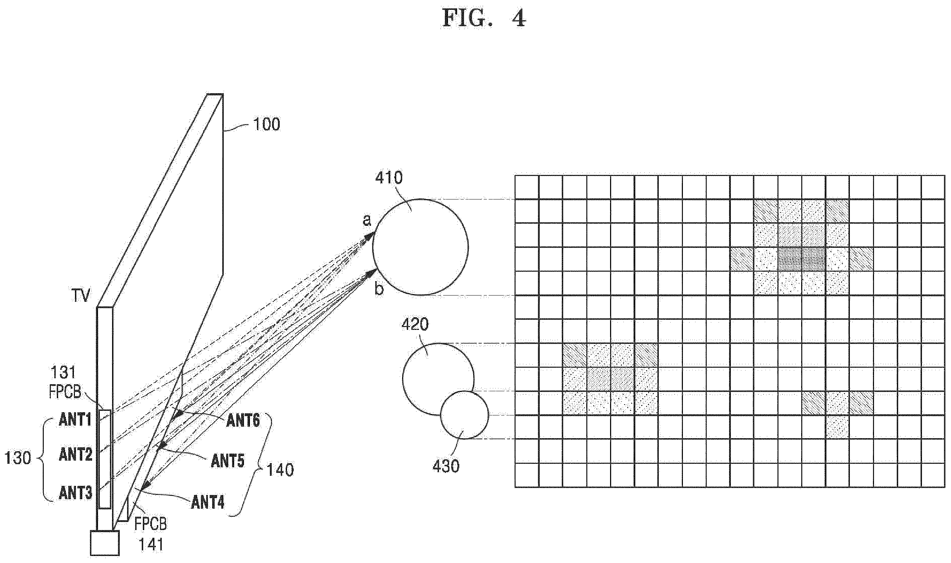

FIG. 4 is a diagram for describing obtaining of three-dimensional information of an object by using an arrangement of a plurality of antennas included in a UWB module, according to an embodiment.

Referring to FIG. 4, a plurality of antennas included in the UWB module 110 may be arranged in the electronic apparatus 100 near the bezel of the front surface of the display. In an embodiment, the plurality of antennas arranged in the electronic apparatus 100 may include FPCB antennas. The FPCB antennas refer to antennas in which electrical characteristics are implemented by forming antenna patterns in thin, flexible FPCBs.

As illustrated in FIG. 4, the FPCBs 131 and 141 may be arranged on the front surface of the display of the electronic apparatus 100 in vertical and horizontal directions. The plurality of antennas ANT1, ANT2, and ANT3 130 in the vertical direction may be vertically arranged on the FPCB 131 vertically arranged on the front surface of the display. In addition, the antennas ANT4, ANT5, and ANT6 140 in the horizontal direction may be horizontally arranged on the FPCB 141 arranged in the horizontal direction on the front surface of the display. The antennas ANT1, ANT2, ANT3 130 in the vertical direction and the antennas ANT4, ANT5, and ANT6 140 in the horizontal direction may be TX antennas, RX antennas, or combined TX/RX antennas.

In an embodiment, at least one transmission antenna and at least one reception antenna may be respectively arranged on the front surface of the display of the electronic apparatus 100 in the vertical and horizontal directions, thereby obtaining three-dimensional information of an object.

In FIG. 4, each of the antennas ANT1, ANT2, and ANT3 130 in the vertical direction may emit a UWB transmission signal. Each of the antennas ANT1, ANT2, and ANT3 130 in the vertical direction may emit a transmission signal at a series of time intervals. The transmission signal emitted by each antenna hits point (a) of a first object 410 and is reflected from point (a) to return to the antennas ANT4, ANT5, and ANT6 140 in the horizontal direction, respectively. Similarly, the transmission signals emitted by the antennas ANT1, ANT2, and ANT3 130 in the vertical direction may be reflected at point (b) of the first object 410 and come back to the antennas ANT4, ANT5, and ANT6 140 in the horizontal direction.

The reflected signals received by the antennas ANT4, ANT5, and ANT6 140 in the horizontal direction may have different reception times, different angles, different magnitudes, according to the positions of a transmission antenna and a reception antenna, a time when the transmission antenna emits a signal, and a distance between each antenna and the first object 410.

In an embodiment, the electronic apparatus 100 may know three-dimensional coordinate values of each antenna included in the UWB module 110. Also, the electronic apparatus 100 may know in advance a propagation speed of a UWB signal, that is, the speed of light.

In an embodiment, the electronic apparatus 100 may obtain a period of time required for reflected radio waves to reach the reception antenna. For example, the electronic apparatus 100 may obtain a period of time taken by a propagation signal emitted from a transmission antenna, to be reflected at a certain point on an object and to reach a reception antenna, by using a method such as Time Difference of Arrival (TDOA) or Time of Arrival (TOA).

In an embodiment, the electronic apparatus 100 may obtain an arrival angle at which a radio signal reaches a reception antenna after being reflected from a certain point of an object. For example, the electronic apparatus 100 may obtain an angle at which the reflection signal arrives, by using a method such as an angle of arrival (AOA) method. The arrival angle may refer to a direction in which a reflected signal is received.

In an embodiment, the electronic apparatus 100 may obtain a distance from an antenna to a certain point of an object and an angle or direction of the certain point, by using coordinate values of each antenna, a moving speed of a radio wave, a period of time required for the radio wave to be received again by a reception antenna after being emitted by a transmission antenna, and a transmission angle and a reception angle of a radio signal. The electronic apparatus 100 may obtain three-dimensional coordinate values of a certain point of an object based on the distance to and angle with respect to the certain point of the object.

In an embodiment, when the certain point of the object moves, the electronic apparatus 100 may obtain movement information of that point. The electronic apparatus 100 may obtain coordinate values of a certain point of an object in three dimensions, a movement direction of the point, and a movement speed of the point, and obtain movement information of the certain point of the object in three dimensions from the obtained information. The electronic apparatus 100 may obtain three-dimensional information of each of the first object 410, the second object 420, and the third object 430 illustrated in FIG. 4.

In an embodiment, the electronic apparatus 100 may display three-dimensional information of each object in a graph or the like. On the right side of FIG. 4, the electronic apparatus 100 displays contour lines of each object obtained by using the three-dimensional information obtained for each of the first object, the second object 420, and the third object 430. The contour lines of the object may be displayed in different values or colors according to a distance between an antenna and the object. Regarding the contour lines shown on the right side of FIG. 4, the contour lines are displayed thicker as the distance between the antenna and the object is smaller, and displayed lighter as the distance between the antenna and the object is greater. In an embodiment, the electronic apparatus 100 may output a graph displaying three-dimensional information for each object, on a display screen.

As such, according to an embodiment, the electronic apparatus 100 may obtain three-dimensional information about each object by using a plurality of antennas arranged in front of the display. Also, the electronic apparatus 100 may display three-dimensional information of an object by a graph.

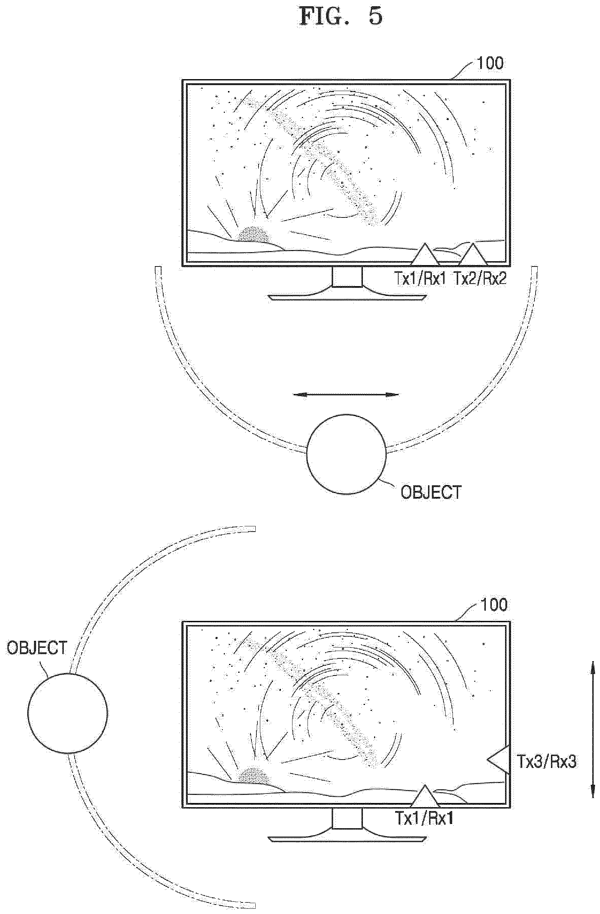

FIG. 5 is a diagram for describing an electronic apparatus detecting an object, according to an embodiment.

The drawing shown at the top of FIG. 5 shows that a movement of an object in a horizontal direction is detected using two antennas arranged in a horizontal direction on a front surface of the electronic apparatus 100.

The UWB module 110 may determine a horizontal distance from the electronic apparatus 100 to the object. The electronic apparatus 100 may measure a distance to the object by using a travel time of a signal.

In an embodiment, at least two antennas may be arranged on the front surface of the electronic apparatus 100 in a horizontal direction. For example, two antennas arranged in a horizontal direction may be both combined transmission/reception antennas. In FIG. 5, two antennas TX1/RX1 and TX2/RX2 are arranged on the front surface of the electronic apparatus 100. The two antennas TX1/RX1 and TX2/RX2 may be used to detect a movement of an object in a horizontal direction. That is, the electronic apparatus 100 may transmit a UWB signal to an object through each of the two antennas TX1/RX1 and TX2/RX2, and receive a signal reflected from the object through each of the two antennas TX1/RX1 and TX2/RX2, respectively.

In an embodiment, the plurality of antennas mounted on the electronic apparatus 100 in a horizontal direction may have different arrangement positions, and may also have different reception times and reception directions of signals from the object. Accordingly, the electronic apparatus 100 may detect a position of the object by considering a time difference in transmitting a signal among the plurality of antennas, an angle difference between the transmitted signals, a time difference and an angle difference between received signals, and the positions of the plurality of antennas. That is, by using at least two combined transmission/reception antennas arranged in a horizontal direction, the electronic apparatus 100 may detect a movement of the object in a horizontal direction and coordinate values of the object in the horizontal direction with respect to the electronic apparatus 100.

The drawing illustrated at the bottom of FIG. 5 illustrates detecting of a movement of an object in a vertical direction by using antennas respectively arranged on the front surface of the electronic apparatus 100 in a horizontal direction and a vertical direction.

In an embodiment, the electronic apparatus 100 may transmit a UWB signal to an object through the antennas TX1/RX1 and TX3/RX3 arranged in a vertical direction, and receive a UWB signal transmitted from the object through the antenna TX1/RX1 and the antenna TX3/RX3. The electronic apparatus 100 may detect a movement of an object in a vertical direction and coordinate values of the object in the vertical direction with respect to the electronic apparatus 100 by using at least two combined transmission/reception antennas arranged up and down in the vertical direction.

The electronic apparatus 100 may detect a movement of an object in a horizontal direction by using a plurality of antennas arranged in a horizontal direction, and may detect a movement of the object in a vertical direction by using a plurality of antennas arranged in a vertical direction. Accordingly, the electronic apparatus 100 may obtain coordinate values and a movement of an object in three dimensions by using a plurality of antennas arranged in a horizontal direction and a plurality of antennas arranged in a vertical direction.

As described above, according to an embodiment, the electronic apparatus 100 may detect a movement of an object in three dimensions by using a plurality of antennas arranged in vertical and horizontal directions.

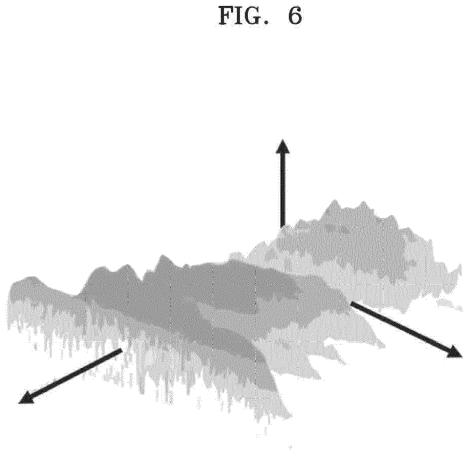

FIG. 6 is a diagram illustrating a signal reflected from an object, according to an embodiment.

Referring to FIG. 6, the electronic apparatus 100 may emit a UWB signal to an object and receive a signal reflected from the object. The electronic apparatus 100 may emit a UWB signal at different times for each antenna by using a plurality of combined transmission/reception antennas respectively arranged in horizontal and vertical directions on a front surface of a display. The emitted UWB signal may be reflected from an object and received through a plurality of combined transmission/reception antennas respectively arranged on the front surface of the display of the electronic apparatus 100 in horizontal and vertical directions.

In an embodiment, reflected signals obtained by the electronic apparatus 100 may include movement information of an object. The reflected signals may include information about a degree or an amount of movement of the object and a direction of the movement of the object in three dimensions.

For example, when the electronic apparatus 100 emits a signal through one transmission antenna TX1 and the signal is reflected from the object and received through reception antennas RX1, RX2, and RX3 at certain, different times, the electronic apparatus 100 may obtain a distance and a direction between each antenna and the object by using a signal received by each reception antenna. The electronic apparatus 100 may obtain a movement of the object in three dimensions based on the distance to and direction of the object.

The electronic apparatus 100 may display the movement of the object by a graph as shown in FIG. 6.

The graph shown in FIG. 6 is a graph in which the movement of the object obtained from a reflected signal received by the electronic apparatus 100 through a reception antenna is displayed as a three-dimensional graph in a time domain. The graph shown in FIG. 6 represents raw data for the movement of the object over time for each of the X-axis, the Y-axis, and the Z-axis.

The electronic apparatus 100 may generate an X-axis signal by connecting peak values of the X-axis of the raw data over time, and generate a Y-axis signal by connecting peak values of the Y-axis of the raw data over time. Similarly, the electronic apparatus 100 may generate a Z-axis signal by connecting peak values of the Z-axis of raw data over time. The electronic apparatus 100 may display the movement of the object by a three-dimensional graph, as shown in the graph of FIG. 6, based on signals obtained by connecting peak values of raw data obtained for each axis.

The movement of the object may include not only large body motions such as walking or running, but also small bio-signal motions such as respiration or heartbeat, when the object is a human. Accordingly, the graph shown in FIG. 6 includes information about motions according to bio-signals and other motions.

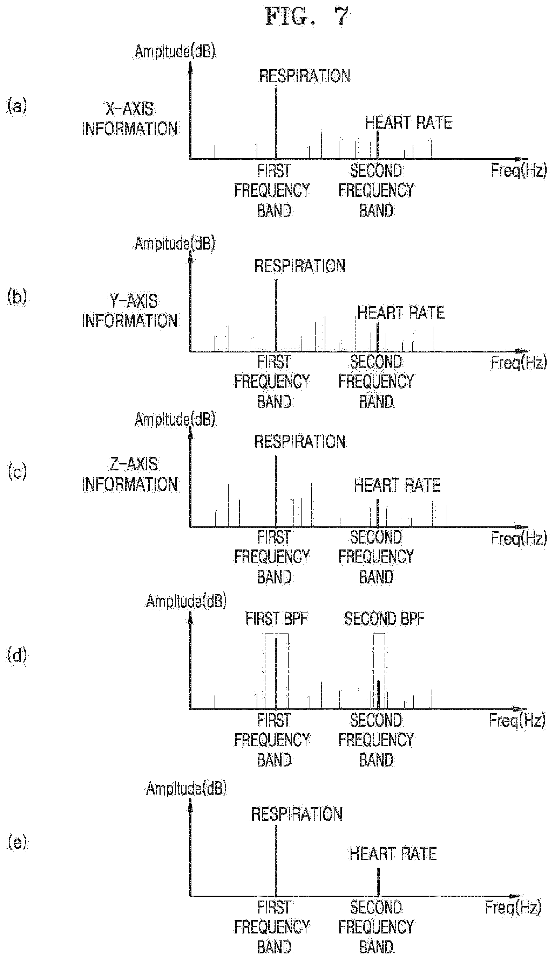

FIG. 7 is a diagram for describing removal of noise from three-dimensional information of an object, according to an embodiment.

Referring to FIG. 7, the electronic apparatus 100 may obtain raw data for each axis from raw data about movement information of an object on three dimensions illustrated in FIG. 6. The electronic apparatus 100 may obtain a signal for each axis in a time domain from three-dimensional information of the object and convert the same into a signal in a frequency domain. The electronic apparatus 100 may convert a signal for each axis in the time domain into a signal in the frequency domain by performing, for example, Fast Fourier Transform (FFT).

(a), (b), and (c) of FIG. 7 show that raw data on the time domain for each of the X-axis, the Y-axis, and the Z-axis is converted into a signal on the frequency domain. As in FIG. 7, a signal in the frequency domain may be expressed as an amplitude value for each frequency.

In an embodiment, the electronic apparatus 100 may filter only signals in a frequency range related to bio-signals. As described above, a UWB reflection signal may include both a signal according to movement of a bio-signal, such as respiration or a heartbeat, and a signal corresponding to large motions of an object.

In an embodiment, the electronic apparatus 100 may obtain only bio-signals from among signals about a movement of the object. To this end, the electronic apparatus 100 may know a frequency range of a bio-signal in advance. Hz is 1/s when expressed as an SI basic unit, and may refer to the number of repetitions per second. Among bio-signals, for example, a respiration rate is a numerical value indicating how many times a person breathes per minute. The normal respiration rate of an adult is 12 times/min to 20 times/min, and when expressed in Hz, it is 0.2 Hz to 0.3 Hz. A heart rate indicates the number of heart beats per unit time, and in general, the heart rate of an adult is 60 times to 100 times per minute, which is 1 Hz to 1.5 Hz.

In an embodiment, the electronic apparatus 100 may know in advance a frequency range corresponding to a respiration rate and a frequency range corresponding to a heart rate. For example, when a first frequency band is a frequency band corresponding to a general respiration rate and a second frequency band is a frequency band corresponding to a general heart rate, the electronic apparatus 100 may filter only signals of the first frequency band and the second frequency band. For example, as shown in (d) of FIG. 7, the electronic apparatus 100 may filter only signals of the first frequency band and the second frequency band by using a band pass filter (BPF) that passes only signals of a certain band.

(e) of FIG. 7 shows a result of filtering a bio-signal by using a BPF suitable for each frequency band. The electronic apparatus 100 may obtain only a bio-signal for a respiration rate by filtering the first frequency band by using a BPF having a certain bandwidth. Also, the electronic apparatus 100 may obtain only a bio-signal for the heart rate by filtering the second frequency band.

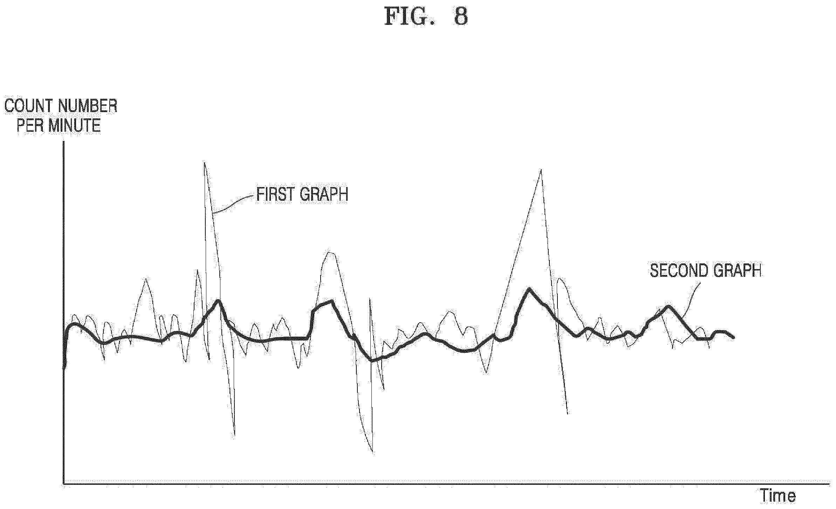

FIG. 8 is a graph showing a bio-signal from which movement noise is removed, according to an embodiment.

The electronic apparatus 100 may obtain information of an object by using a UWB signal. The information of the object may include a signal according to a movement of the object. The movement of the object may include various large and small motions in addition to motions of bio-signals such as a respiration rate or heart rate.

A first graph shown in FIG. 8 is a graph displaying the heart rate of a moving object. The first graph shows the heart rate of the object over time, and includes noise according to other movements in addition to the movement according to the heart rate.

In an embodiment, the electronic apparatus 100 may obtain a graph of the heart rate of the object by using a UWB signal. A second graph shown in FIG. 8 is a graph showing the heart rate of the object over time, which is obtained using a UWB signal.

In order to obtain the second graph, the electronic apparatus 100 may obtain a signal for each axis with respect to a movement of the object on a time domain and convert the signal for each axis into a signal on a frequency domain. The electronic apparatus 100 may filter only signals having a certain frequency band among signals in the frequency domain. That is, the electronic apparatus 100 may remove noise caused by motions other than motions according to the heart rate, by filtering only signals in an Hz range suitable for the heart rate by using a BPF having a certain bandwidth. The electronic apparatus 100 may obtain the second graph by converting a filtered signal back into a signal on the time domain.

In the second graph, noises according to other motions of the object than the heart rate are removed.

As described above, according to the embodiment, by using a UWB signal, the electronic apparatus 100 may detect only the motion according to a certain bio-signal among motions of the object.

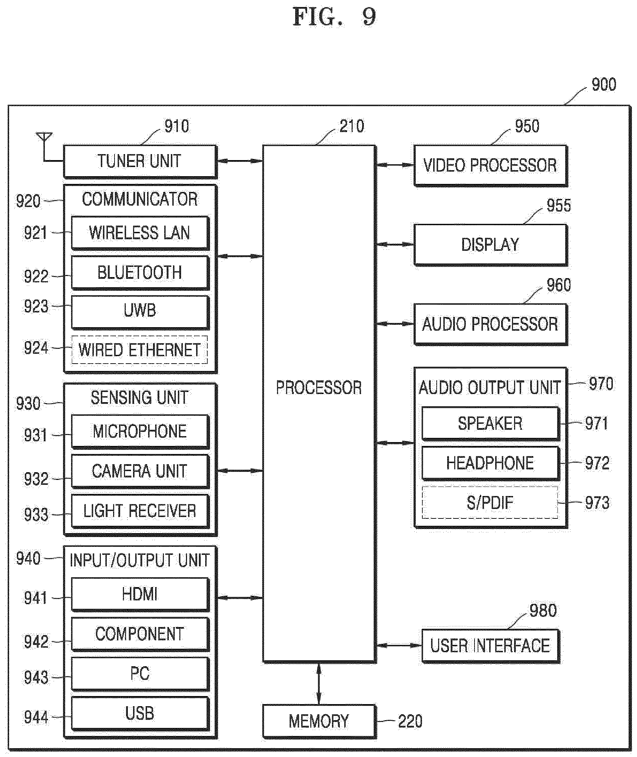

FIG. 9 is an internal block diagram of an electronic apparatus according to an embodiment.

An electronic apparatus 900 may be an image display apparatus. The image display apparatus may be a digital TV capable of receiving digital broadcasting, but is not limited thereto, and may be implemented in various types of electronic apparatuses.

The electronic apparatus 900 of FIG. 9 may include the components of the electronic apparatus 200 of FIG. 2. Referring to FIG. 9, the electronic apparatus 900 may include a tuner unit 910, a communicator 920, a sensing unit 930, an input/output unit 940, and a video processor 950, a display 955, an audio processor 960, an audio output unit 970, and a user interface 980, in addition to the processor 210 and the memory 220.

The tuner unit 910 may tune and select only a frequency of a channel to be received by the electronic apparatus 900 from among numerous radio wave components through amplification, mixing, resonance, etc. of broadcasting contents received in a wired or wireless manner. The content received through the tuner unit 910 is decoded and separated into audio, video and/or additional information. The separated audio, video and/or additional information may be stored in the memory 220 under the control of the processor 210.

The communicator 920 may connect the electronic apparatus 900 to a peripheral device, an external device, or a server under the control of the processor 210. The electronic apparatus 900 may download a program or an application required by the electronic apparatus 900 from an external device or server through the communicator 920 or may perform web browsing for the same. Also, the communicator 920 may receive content from an external device.

The communicator 920 may include at least one of a wireless LAN 921, Bluetooth 922, UWB 923, and wired Ethernet 924 corresponding to the performance and structure of the electronic apparatus 900.

The communicator 920 may receive a control signal through a control device (not shown) such as a remote controller under the control of the processor 210. The control signal may be implemented as a Bluetooth type, an RF signal type, or a Wi-Fi type. The communicator 920 may further include other short-range communication (e.g., near field communication (NFC), not shown) in addition to the Bluetooth 922. The Bluetooth 922 may include a Bluetooth Low Energy (BLE) communication module. The BLE communication module may communicate with nearby user terminals (not shown) through a BLE communication scheme.

In an embodiment, the UWB 923 may include an ultra-wideband (UWB) module. The UWB module may emit a signal to nearby objects by using a very low spectral power density over a very wide frequency band by using very narrow pulses of several nanoseconds or picoseconds, and measure a distance to the object and a position of the object from a signal reflected from the object. In an embodiment, the UWB module may include a plurality of antennas respectively arranged in vertical and horizontal directions on a front surface of the display 955. The UWB module may accurately measure three-dimensional coordinate values, a movement degree, a movement direction, etc. of an object by using the plurality of antennas arranged in horizontal and vertical directions.

The sensing unit 930 may detect a user's voice, a user's image, or a user's interaction, and may include a microphone 931, a camera unit 932, and a light receiver 933. The microphone 931 may receive a user's uttered voice and convert the received voice into an electrical signal and output the same to the processor 210. The camera unit 932 may include a sensor (not shown) and a lens (not shown), and may capture an image formed on a screen. The light receiver 933 may receive an optical signal (including a control signal). The light receiver 933 may receive an optical signal corresponding to a user input (e.g., a touch, a pressing, a touch gesture, a voice, or a motion) from a control device (not shown) such as a remote control or a mobile phone. A control signal may be extracted from the received optical signal under the control of the processor 210.

The input/output unit 940 may receive video (e.g., a video signal or a still image signal), audio (e.g., a voice signal or a music signal), and additional information such as metadata, from an external device of the electronic apparatus 900 under the control of the processor 210. The metadata may include HDR information about content, a description of the content, a title of the content, a storage position of the content, and the like. The input/output unit 940 may include one of a High-Definition Multimedia Interface port 941, a component jack 942, a PC port 943, and a USB port 944. The input/output unit 940 may include a combination of the HDMI port 941, the component jack 942, the PC port 943, and the USB port 944.

The video processor 950 processes image data to be displayed by the display 955 and may perform various image processing operations such as decoding, rendering, scaling, noise filtering, frame rate conversion, and resolution conversion on the image data.

The display 955 may display, on a screen, content received from a broadcasting station or from an external server or an external storage medium. The content may include, as a media signal, a video signal, an image, a text signal, or the like. Also, the display 955 may display a video signal or an image received through the HDMI port 941 on the screen.

When the display 955 is implemented as a touch screen, the display 955 may be used as an input device in addition to an output device. For example, the display 955 may include at least one of a liquid crystal display, a thin film transistor-liquid crystal display, an organic light-emitting diode, a flexible display, a 3D display, and an electrophoretic display. Also, depending on the implementation form of the display 955, two or more displays 955 may be included.

The audio processor 960 processes audio data. The audio processor 960 may perform various processes such as decoding or amplifying audio data and filtering noise.

The audio output unit 970 may output audio included in content received through the tuner unit 910, audio input through the communicator 920 or the input/output unit 940, and audio stored in the memory 220 under the control of the processor 210. The audio output unit 970 may include at least one of a speaker 971, a headphone output terminal 972, and a Sony/Philips Digital Interface (S/PDIF) output terminal 973.

The user interface 980 may receive a user input for controlling the electronic apparatus 900. The user interface 980 may include various types of user input devices including a touch panel that detects a user's touch, a button that receives a user's push operation, a wheel that receives a user's rotation operation, a keyboard, and a dome switch, a microphone for voice recognition, a motion sensor for sensing motion, and the like, but is not limited thereto. Also, when the electronic apparatus 900 is manipulated by a remote controller (not shown), the user interface 980 may receive a control signal received from the remote controller.

The processor 210 may control the overall operation of the electronic apparatus 900.

In an embodiment, the processor 210 may obtain three-dimensional information of an object based on a UWB signal transmitted or received by a UWB module. In an embodiment, the processor 210 may obtain three-dimensional information of an object by using a plurality of antennas included in the UWB module. In an embodiment, the processor 210 may obtain three-dimensional information of an object by using at least one of positions of a transmission antenna and a reception antenna, an emission time of a UWB transmission signal, a reception time of a UWB reflection signal, and magnitudes and angles of the UWB transmission signal and the reflection signal.

In an embodiment, the processor 210 may obtain a difference in emission times of a UWB transmission signal between a plurality of antennas, a difference in reception times of a UWB reflection signal between a plurality of antennas, a difference in magnitudes and angles of UWB transmission signals and UWB reception signals between the plurality of antennas, and a difference in positions of the plurality of antennas, and obtain the three-dimensional information of the object based on the differences. In an embodiment, the three-dimensional information of the object may include at least one of position information, direction information, movement amount information, and movement speed information of the object.

In an embodiment, the processor 210 may obtain, from the three-dimensional information of the object, a bio-signal from which movement noise is removed. The processor 210 may obtain a signal on a frequency domain from the three-dimensional information of the object. That is, the processor 210 may obtain a signal of each of the X-axis, the Y-axis, and the Z-axis from the three-dimensional information of the object, and convert each signal of the X-axis, Y-axis, and Z-axis into a frequency domain signal. The processor 210 may filter signals having a certain frequency range from signals in the frequency domain and obtain only bio-signals of a desired frequency band by inversely transforming the filtered signals into a time domain. The processor 210 may obtain a biosignal including at least one of heart rate and respiration

In an embodiment, the processor 210 may detect a change in a bio-signal, the change being greater than or equal to a reference value. The processor 210 may generate notification information notifying a change in a bio-signal, the change being greater than or equal to a reference value, and output the notification information. The notification information may include at least one of an audio signal and a video signal. The processor 210 may output notification information through the audio output unit 970 or the display 955 of the electronic apparatus 900, or may transmit notification information to an external user terminal through the communicator 920.

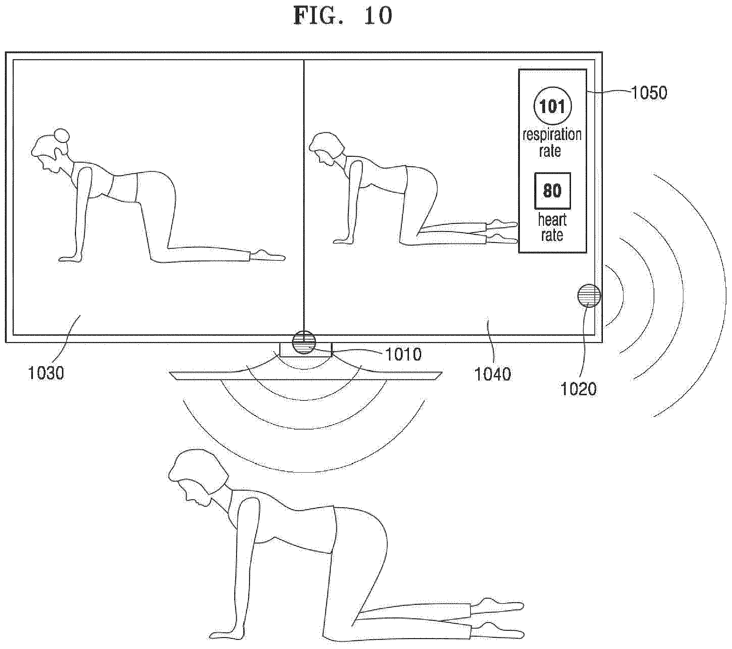

FIG. 10 is a diagram for describing an electronic apparatus outputting comparison content, according to an embodiment.

Referring to FIG. 10, the electronic apparatus 100 may be connected to a source device (not shown). The source device may include at least one of a personal computer (PC), a DVD player, a video game console, a set-top box, an AV receiver, a cable receiver or a satellite broadcast receiver, or an Internet receiver that receives content from an Over The Top (OTT) service provider or an Internet Protocol Television (IPTV) service provider. The electronic apparatus 100 may be connected to a source device, receive content stored in a medium, and output the content through a display. Alternatively, the electronic apparatus 100 may stream or download content from a server (not shown) through the source device. Various types of content created by content providers such as terrestrial broadcasting stations, cable broadcasting stations, OTT service providers, and IPTV service providers may be stored in the server. Content may include one or more of a video signal, an audio signal, and a text signal.

In an embodiment, the electronic apparatus 100 may display comparison content 1030 on a screen.

In an embodiment, the electronic apparatus 100 may provide a multi-streaming service to provide users with more diverse content experiences. The multi-streaming service may refer to a service in which the electronic apparatus 100 receives multi-streams, processes the received multi-streams, and provides different contents in a plurality of areas of a display screen. A multi-streaming service may also be referred to as a multi-view service or a multi-screen service.

The electronic apparatus 100 may provide content in a multi-view mode by dividing a display into a plurality of screens and displaying different contents on each of the divided screens. In the multi-view mode, each divided screen of the display may also be referred to as a partial screen.

Contents displayed in the multi-view mode may include one or more of broadcasting content received via a direct RF signal from a broadcasting station, broadcasting content received through an external source, and content received from a content providing server through the Internet.

Also, the electronic apparatus 100 may obtain a real-time image 1040 of a user by using a camera (not shown) and output the same on a screen. The camera may be fixedly mounted inside the electronic apparatus 100, or may be a camera included in a terminal separate from the electronic apparatus 100, such as a mobile phone connected to the electronic apparatus 100 through a wired or wireless communication network.

However, this is an example, and the electronic apparatus 100 may not use a camera. For example, the electronic apparatus 100 may generate a contour graph of a three-dimensional shape of an object based on three-dimensional information obtained with respect to the object through a UWB signal and output the graph on a screen together with comparison content.

In FIG. 10, a UWB module may be mounted in the electronic apparatus 100. The UWB module may include a plurality of antennas 1010 arranged in a horizontal direction and a plurality of antennas 1020 arranged in a vertical direction. The electronic apparatus 100 may obtain three-dimensional information of an object by using the plurality of antennas 1010 and 1020.

In an embodiment, the electronic apparatus 100 may obtain, from the three-dimensional information of the object, a bio-signal from which movement noise is removed. The electronic apparatus 100 may obtain a three-dimensional signal with respect to a movement of an object by using a UWB signal and obtain a signal for each axis from the three-dimensional signal. That is, the electronic apparatus 100 may obtain a signal about the movement of an object for each of the X-axis, Y-axis, and Z-axis, and convert the signal into a signal on the frequency domain. In the frequency domain, a signal for the movement of the object may be expressed as an amplitude according to a frequency. In an embodiment, the electronic apparatus 100 may filter only signals of a frequency band related to bio-signals, from signals in the frequency domain. For example, the electronic apparatus 100 may filter only signals of a frequency band related to a respiration rate or a heart rate by using a certain BPF. The electronic apparatus 100 may obtain a bio-signal from which movement noise is removed, by converting a filtered signal into a signal on a time domain again.

The electronic apparatus 100 may output the bio-signal to a screen. As illustrated in FIG. 10, the electronic apparatus 100 may output a bio-signal 1050 such as an object's respiration rate or heart rate on a screen. A user may copy a motion by viewing the comparison content 1030 output on a screen of the electronic apparatus 100 and check the bio-signal 1050 of his or her own in real time while exercising.

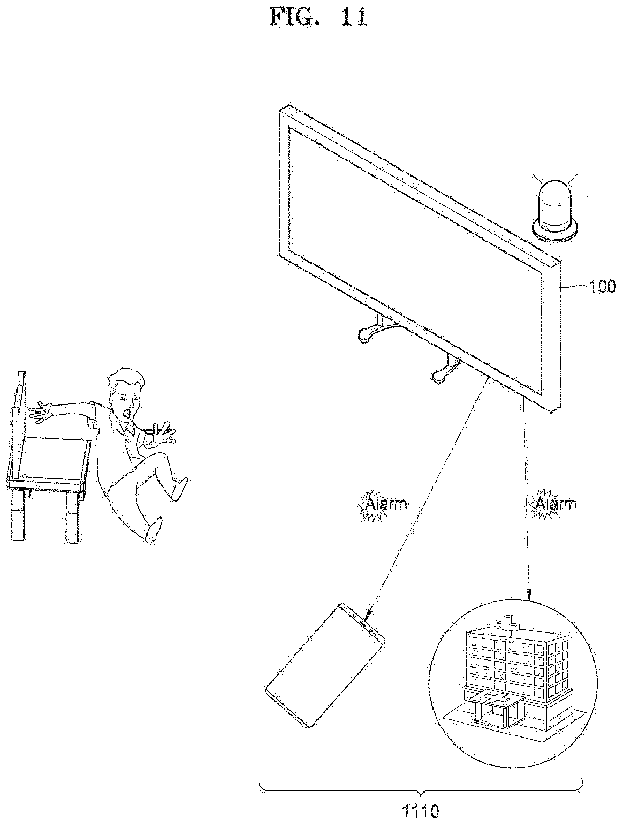

FIG. 11 is a diagram for describing a case where the electronic apparatus 100 has detected an abnormal change in a bio-signal, according to an embodiment.

Referring to FIG. 11, the electronic apparatus 100 may obtain a bio-signal of an object from three-dimensional information of the object. The electronic apparatus 100 may periodically or continuously monitor whether the heart rate or respiration rate of the object is within a certain reference range. For example, in the graph of FIG. 8, the electronic apparatus 100 may detect that an emergency situation has occurred in the object when the Y-axis value of the second graph is out of a predetermined reference range, for example, when the heart rate rapidly drops to 30 per minute. The electronic apparatus 100 may generate notification information notifying a change in a bio-signal greater than or equal to a reference value.

For example, the electronic apparatus 100 may output a siren sound indicating an emergency through a speaker or output a red blinking light through a display. And/or the electronic apparatus 100 may transmit an emergency notification signal to an external user terminal 1110 through a communication network (not shown). The external user terminal 1110 may be a terminal of a user pre-registered in the electronic apparatus 100 or a terminal operated by a pre-registered institution such as a hospital or fire station.

As described above, according to an embodiment, the electronic apparatus 100 may detect a bio-signal of the object in real time and notify the surroundings when a sudden change in the bio-signal is detected.

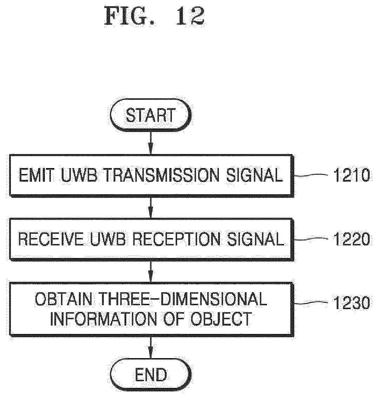

FIG. 12 is a flowchart of an operating method of an electronic apparatus, according to an embodiment.

The electronic apparatus includes a UWB module, and the UWB module has UWB antennas arranged along horizontal and vertical axes of a display. The electronic apparatus may emit a UWB transmission signal through a UWB transmission antenna (operation 1210).

The electronic apparatus may receive a reflected UWB reflection signal through a UWB reception antenna (operation 1220).

The electronic apparatus may obtain three-dimensional information from an object based on the UWB transmission signal and the UWB reflection signal (operation 1230). The electronic apparatus may obtain three-dimensional information of the object by using at least one of positions of a plurality of UWB antennas, a time when the UWB transmission signal is emitted, a time when the UWB reflection signal is received, and magnitudes and angles of the UWB transmission signal and the UWB reflection signal.

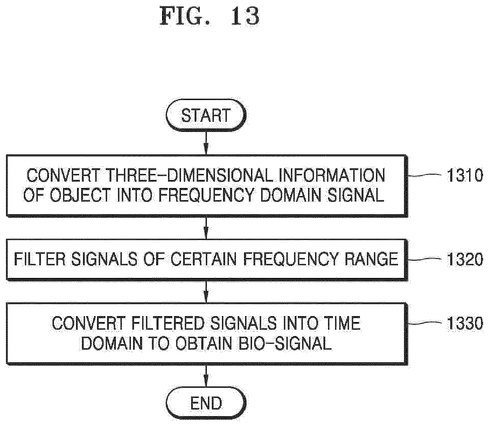

FIG. 13 is a flowchart of an operating method of an electronic apparatus, according to an embodiment.

Referring to FIG. 13, after obtaining three-dimensional information of an object by using the method of FIG. 12, the electronic apparatus may obtain a bio-signal from which movement noise is removed, from the three-dimensional information of the object.

In detail, the electronic apparatus may convert the three-dimensional information of the object into a signal on a frequency domain (operation 1310). As the three-dimensional information of the object is a signal in a time domain, the electronic apparatus may separately obtain signals for each axis, that is, the X-axis, the Y-axis, and the Z-axis, from the three-dimensional information of the object. The electronic apparatus may convert a signal for each axis in the time domain into a signal in the frequency domain by performing, for example, Fast Fourier Transform (FFT) on each axis signal.

The electronic apparatus may filter signals in a certain frequency range among signals in the frequency domain (operation 1320). A memory of the electronic apparatus may already store information about bio-signals having a period within a certain range, such as a heart rate or a respiration rate. The electronic apparatus may filter only signals of a frequency band corresponding to a certain bio-signal through BPF.

The electronic apparatus may inversely transform the filtered signal into a signal in the time domain to obtain a bio-signal in the time domain (operation 1330). The bio-signal converted into the time domain may include only a pure bio-signal from which movement noise is removed, from among the three-dimensional information of the object.

The electronic apparatus may obtain a bio-signal from the three-dimensional information of the object periodically, continuously, or at random intervals.

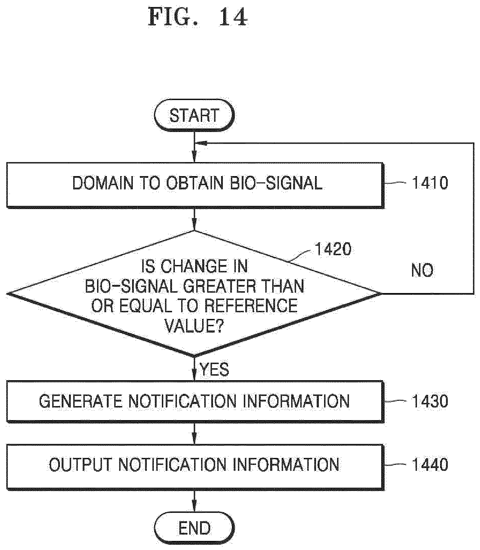

FIG. 14 is a flowchart of an operating method of an electronic apparatus, according to an embodiment.

Referring to FIG. 14, the electronic apparatus may monitor whether there is a change in a bio-signal of an object (operation 1410). For example, the electronic apparatus may newly obtain a bio-signal of an object periodically, continuously, or at random intervals, and determine whether there is a change in the bio-signal.

In an embodiment, the electronic apparatus may generate notification information when a change in a bio-signal is detected and when the change is greater than or equal to a certain reference value, the electronic apparatus may generate notification information (operation 1430). The notification information may include at least one of an audio signal and a video signal.

In an embodiment, the electronic apparatus may generate notification information even when an abrupt change in a movement of an object is detected and the change is greater than or equal to a certain reference value.

In an embodiment, the electronic apparatus may detect whether an emergency situation has occurred, such as when an object falls or does not breathe, based on at least one of a change in a movement of the object and a change in a bio-signal.

In this case, the electronic apparatus may output notification information (operation 1440). The notification information may include at least one of an audio signal and a video signal. The electronic apparatus may output an audio signal through a speaker attached to the electronic apparatus or output a video signal through a display of the electronic apparatus. Alternatively, the electronic apparatus may transmit notification information to a remote device such as an external user terminal through a communicator.

An electronic apparatus and an operating method therefor, according to some embodiments, may also be realized in a form of a recording medium including instructions executable by a computer, such as a program module executed by a computer. A computer-readable recording medium may be an arbitrary available medium accessible by a computer, and may be any one of volatile, nonvolatile, separable, and non-separable media. Also, examples of the computer-readable recording medium may include a computer storage medium and a communication medium. Examples of the computer storage medium include volatile, nonvolatile, separable, and non-separable media realized by an arbitrary method or technology for storing information about a computer-readable command, a data structure, a program module, or other data. The communication medium may include a computer-readable command, a data structure, a program module, other data of a modulated data signal, such as carrier waves, or other transmission mechanisms, and may be an arbitrary information transmission medium.

Also, "unit" in the present specification may be a hardware component such as a processor or a circuit, and/or a software component executed by a hardware component such as a processor.

In addition, the electronic apparatus and the operating method therefor, according to the embodiments of the present disclosure described above, may be implemented by a computer-readable recording medium having recorded thereon a program for executing the operating method of the electronic apparatus, the operating method including emitting a UWB transmission signal by using a UWB communication module including a plurality of antennas, receiving a UWB reflection signal reflected from an object, and obtaining three-dimensional information of the object based on the UWB transmission signal and the UWB reflection signal, wherein the plurality of antennas are respectively arranged in a vertical direction and a horizontal direction on a front surface of a display of the electronic apparatus.

While the present disclosure has been particularly shown and described with reference to embodiments thereof, it will be understood by those of ordinary skill in the art that various changes in form and details may be made therein without departing from the spirit and scope of the present disclosure as defined by the appended claims. The embodiments should be considered in a descriptive sense only and not for purposes of limitation. For example, each element described as a single type may be distributed, and similarly, elements described to be distributed may be combined.

## Claims

1. An operating method of an electronic apparatus, the operating method being performed using a UWB module including a plurality of antennas, and comprising:
emitting a UWB transmission signal;
receiving a UWB reflection signal reflected from an object; and
obtaining three-dimensional information of the object based on the UWB transmission signal and the UWB reflection signal,
wherein the plurality of antennas are respectively arranged in a vertical direction and a horizontal direction on a front surface of a display of the electronic apparatus.

2. The operating method of claim 1, wherein the obtaining of the three-dimensional information of the object comprises obtaining the three-dimensional information of the object by using at least one of positions of the plurality of antennas, an emission time of the UWB transmission signal, a reception time of the UWB reflection signal, and magnitudes and angles of the UWB transmission signal and the reflection signal.

3. The operating method of claim 2, wherein the obtaining of the three-dimensional information of the object comprises obtaining at least one of a difference in emission times of the UWB transmission signal between the plurality of antennas, a difference in reception times of the UWB reflection signal between the plurality of antennas, a difference in magnitudes and angles of the UWB transmission signal and the UWB reception signal between the plurality of antennas, and a difference in the positions of the plurality of antennas.

4. The operating method of claim 1, wherein the three-dimensional information of the object comprises at least one of position information, direction information, movement amount information, and movement speed information of the object.

5. The operating method of claim 1, further comprising obtaining a bio-signal from which movement noise is removed, from the three-dimensional information of the object,
wherein the obtaining of the bio-signal from which the movement noise is removed comprises:
obtaining a signal on a frequency domain, from the three-dimensional information of the object;
filtering a signal having a certain frequency range, from the signal on the frequency domain; and
inversely transforming the filtered signal having the certain frequency range into a time domain.

6. The operating method of claim 5, wherein the obtaining of the signal on the frequency domain comprises:
obtaining a signal of each of an X-axis, a Y-axis, and a Z-axis from the three-dimensional information of the object; and
converting the signal of each of the X-axis, the Y-axis, and the Z-axis into a frequency domain signal.

7. The operating method of claim 5, wherein the bio-signal comprises at least one of a heart rate and respiration.

8. The operating method of claim 5, further comprising outputting the bio-signal.

9. The operating method of claim 5, further comprising:
detecting a change in the bio-signal, the change being greater than or equal to a reference value;
generating notification information in response to the change in the bio-signal, the change being greater than or equal to the reference value; and
outputting the notification information.

10. The operating method of claim 9, wherein the notification information comprises at least one of an audio signal and a video signal, and
the outputting of the notification information comprises at least one of outputting the notification information through the electronic apparatus and transmitting the notification information to an external user terminal through a communication network.

11. An electronic apparatus comprising:
a UWB module comprising a plurality of antennas;
a memory storing one or more instructions; and
a processor configured to execute the one or more instructions stored in the memory,
wherein the UWB module transmits a UWB transmission signal to an object and receives a UWB reflection signal reflected from the object, and
the processor executes the one or more instructions to obtain three-dimensional information of the object based on the UWB transmission signal and the UWB reflection signal, and the plurality of antennas are respectively arranged in a vertical direction and a horizontal direction on a front surface of the electronic apparatus.

12. The electronic apparatus of claim 11, wherein the processor executes the one or more instructions to obtain the three-dimensional information of the object by using at least one of positions of the plurality of antennas, an emission time of the UWB transmission signal, a reception time of the UWB reflection signal, and magnitudes and angles of the UWB transmission signal and the reflection signal.

13. The electronic apparatus of claim 12, wherein the processor executes the one or more instructions to obtain at least one of a difference in emission times of the UWB transmission signal between the plurality of antennas, a difference in reception times of the UWB reflection signal between the plurality of antennas, a difference in magnitudes and angles of the UWB transmission signal and the UWB reception signal between the plurality of antennas, and a difference in the positions of the plurality of antennas.

14. The electronic apparatus of claim 11, wherein the three-dimensional information of the object comprises at least one of position information, direction information, movement amount information, and movement speed information of the object.

15. A computer-readable recording medium having recorded thereon a program for executing an operating method of an electronic apparatus, the operating method being performed using a UWB communication module including a plurality of antennas, and comprising:
emitting a UWB transmission signal;
receiving a UWB reflection signal reflected from an object; and
obtaining three-dimensional information of the object based on the UWB transmission signal and the UWB reflection signal,
wherein the plurality of antennas are respectively arranged in a vertical direction and a horizontal direction on a front surface of a display of the electronic apparatus.
